# EUROPEAN PATENT APPLICATION

(11) **EP 2 624 161 A1**
(43) Date of publication of application: **07.08.2013**
(21) Application number: 11828659.0
(22) Date of filing: 24.08.2011
(51) Int. Cl.: G06F 21/20, A61B 6/00, G03B 42/04, H04N 5/225

(54) **ELECTRONIC CASSETTE AND ELECTRONIC CASSETTE DEVICE**

(30) Priority: 28.09.2010 JP 2010217402
(71) Applicant: FUJIFILM Corporation, Tokyo 106-8620 (JP)
(72) Inventor: KITANO, Kouichi, Ashigarakami-gun Kanagawa 258-8538 (JP)
(74) Representative: Klunker . Schmitt-Nilson . Hirsch
(86) International application number: PCT/JP2011/069012
(87) International publication number: WO 2012/043098

(57) **Abstract**

An electronic cassette and a radiation imaging system capable of preventing information leakage from a universal memory device and improving usability. A USB memory device 30 incorporates a flash memory 32 and a memory controller 33. An access lock is placed on a data storage area 32b of the flash memory 32. When the USB memory device 30 is attached to an electronic cassette 11, a cassette controller 55 controls a USB controller 60 to input an unlock password to the USB memory device 30. The memory controller 33 unlocks the access lock on the data storage area 32b when the unlock password matches a password stored in a program area 32a in advance.

## Description

### TECHNICAL FIELD

The present invention relates to an electronic cassette and an electronic cassette apparatus for receiving radiation passed through a subject and detecting a radiation image.

### BACKGROUND ART

In the medical field, an X-ray imaging apparatus using radiation, for example, X-rays, to perform diagnostic imaging is known. The X-ray imaging apparatus is provided with an X-ray source for emitting X-rays toward a subject and an X-ray image detection device for receiving the X-rays passed through the subject and detecting an X-ray image. An X-ray image detection device employing an FPD (Flat Panel Detector) has been practically used. In the FPD, an X-ray sensitive layer is disposed over a TFT (Thin Film Transistor) active matrix substrate. The FPD converts intensity distribution of the X-rays that carry image information of a subject (for example, a patient) of an X-ray examination into digital image data.

There are stationary type X-ray image detectors and mobile type X-ray image detectors (electronic cassettes). In the stationary type X-ray image detector, the FPD is attached to an upright table used for imaging a subject in a standing position or a recumbent table used for imaging a subject in a lying position. In the mobile type X-ray image detector, the FPD is incorporated in a flat, substantially rectangular cassette-type housing.

As disclosed in patent document 1, an electronic cassette provided with a memory attachment portion to which a universal memory device such as a USB (Universal Serial Bus) memory device is detachably attached is known. Image data is stored in the universal memory device. Thereby, the image data is taken out from the electronic cassette using the universal memory device when, for example, a communicator used for data transmission provided in the electronic cassette is out of order.

The electronic cassette is often used outside of an imaging room. When it is difficult to move a patient to an imaging room, imaging is performed at the bedside of the patient in a lying position. In this case, the electronic cassette is used in combination with a medical equipment carrier that is a mobile X-ray generating device in which an X-ray source is mounted on a cart.

A medical equipment carrier of a relatively new type often has a communication function to communicate with an electronic cassette and a storing function to store images received from the electronic cassette using the communication function. However, a medical equipment carrier of an old type for a film cassette, an IP (imaging plate) cassette, or the like, are not provided with the communication function and the storing function. In this case, it is convenient if the universal memory device has a function to store images as disclosed in the patent document 1, because the images can be taken out from the electronic cassette through the universal memory device.

### PRIOR ART DOCUMENTS

### PATENT DOCUMENTS

PATENT DOCUMENT 1: Patent Laid-Open Publication No. 2009-45435

### SUMMARY OF THE INVENTION

### Problems to be Solved by the Invention

However, the universal memory device is likely to be lost because it is small. An image of a patient is personal information and leakage to a third party must be avoided. The patent document 1 does not disclose measures to prevent information leakage through the universal memory device.

To prevent the information leakage, a universal memory device having an access lock function may be used. The access lock is unlocked by inputting a password. However, the use of such universal memory device requires inputting an unlock password to unlock the access lock.

As a method for inputting the unlock password, the electronic cassette may be provided with an external interface such as a connector for connecting a keyboard, and the unlock password is inputted through the keyboard. However, this method is bothersome and reduces usability because attachment and removal of the keyboard are necessary. The method for manually inputting the unlock password may cause time delay if an operator forgets the unlock password and hence is not suitable for the electronic cassette that is often used in emergency.

An object of the present invention is to prevent information leakage when a universal memory device is lost and to improve usability.

### Means for Solving the Problems

An electronic cassette of the present invention, to which a memory device is detachably attached, is provided with an image detector, an unlock password storage, and an unlock password input section. Writing to the memory device is allowed with an access lock unlocked by inputting an unlock password. The image detector detects radiation passed through a subject and produces image data. The unlock password storage stores the unlock password. The unlock password input section reads the unlock password from the unlock password storage and inputs the unlock password to the memory device, after the memory device is attached.

It is preferable that the electronic cassette further comprises a controller. The controller determines the memory device, with the access lock unlocked by inputting the unlock password, as a matching memory device and allows writing of the image data. The controller inhibits writing of the image data to a non-matching memory device that is not the matching memory device.

It is preferable that the electronic cassette further comprises a password changer for changing the unlock password, stored in the unlock password storage, based on a change command inputted from an external device.

It is preferable that the electronic cassette further comprises a data reader for reading data, related to imaging conditions of radiation imaging, from the matching memory device.

It is preferable that the electronic cassette further comprises a communicator for communicating data with an external device. In this case, it is preferable that the controller allows the communicator to operate only when the matching memory device is attached to the memory attachment portion.

It is preferable that the memory device is a USB memory device.

An electronic cassette apparatus of the present invention comprises a memory device, a memory attachment portion, an image detector, an unlock password storage, and an unlock password input section. Writing to the memory device is allowed with an access lock unlocked by inputting an unlock password. The memory device is attached to the memory attachment portion in a detachable manner. The image detector detects radiation passed through a subject and produces image data. The unlock password storage stores the unlock password. The unlock password input section reads the unlock password from the unlock password storage and inputs the unlock password to the memory device, after the memory device is attached to the memory attachment portion.

### Effect of the Invention

According to the present invention, the unlock password input section is provided. The unlock password input section reads the unlock password from the unlock password storage and inputs the unlock password to the memory device when the memory device is attached to the memory attachment portion. Hence, the information leakage is prevented when the universal memory device is lost and the usability is improved.

### BRIEF DESCRIPTION OF DRAWINGS

Fig. 1 is an explanatory view illustrating a configuration of an X-ray imaging system.
Fig. 2 is a perspective view illustrating an electronic cassette apparatus.
Fig. 3 is a block diagram illustrating a configuration of the electronic cassette apparatus.
Fig. 4 is a block diagram illustrating a configuration of an FPD.
Fig. 5 is a flowchart illustrating imaging processes carried out by the electronic cassette apparatus.

### MODE FOR CARRYING OUT THE INVENTION

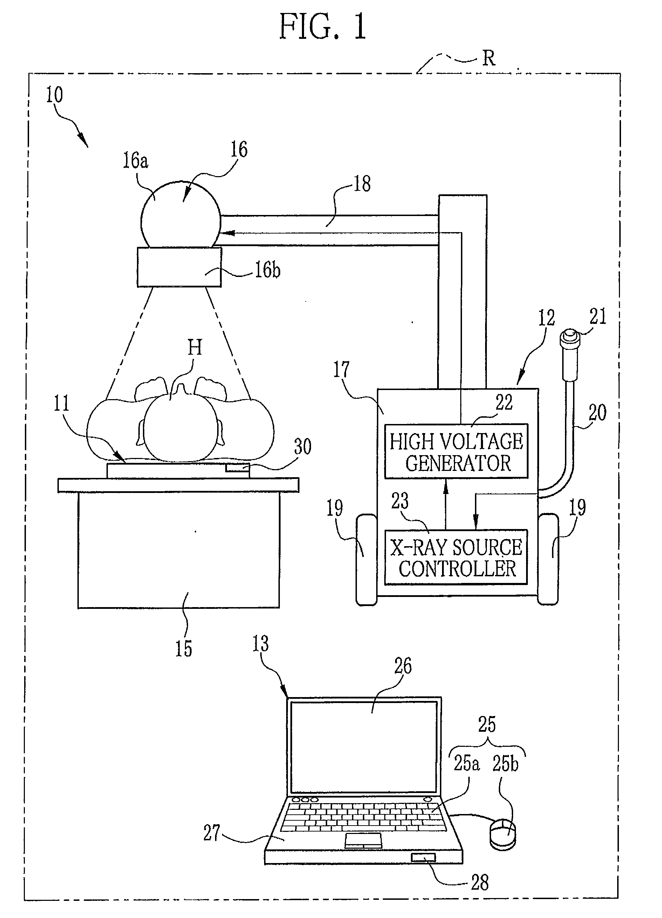

In Fig. 1, an X-ray imaging system 10 is provided with an electronic cassette 11, a medical equipment carrier 12, and a console 13. The X-ray imaging system 10 is used in a room R in which external wireless communication is disabled. A subject H is imaged in a lying state on a table 15. The electronic cassette 11 is placed between the table 15 and the subject H.

The medical equipment carrier 12 is composed of an X-ray source 16, a housing 17, and a movable arm 18. The movable arm 18 connects the housing 17 and the X-ray source 16. The medical equipment carrier 12 is an old type medical equipment carrier not provided with a communication function to communicate with the electronic cassette 11, a saving function to save X-ray images, and a display function to display the X-ray images. An operation panel (not shown) is provided on a top surface of the housing 17. Castors for moving are attached close to the bottom of the housing 17. An emission switch 21 for inputting an emission start signal is connected to the housing 17 through a cable 20.

The movable arm 18 allows the X-ray source 16 to be moved to a desired position suitable for a region to be examined of the subject H. The X-ray source 16 can be put away so as not to obstruct a doctor or technologist conducting diagnosis. The X-ray source 16 is provided with an X-ray tube 16a for emitting X-rays and a collimator (beam limiting device) 16b for restricting an X-ray emission range (X-ray field). The housing 17 of the medical equipment carrier 12 incorporates a high voltage generator 22 and an X-ray source controller 23.

Imaging conditions such as a tube voltage, a tube current, and exposure time are inputted to the X-ray source controller 23 through an operation panel (not shown). The X-ray source controller 23 sends the inputted imaging conditions to the high voltage generator 22. The emission switch 21 is connected to the X-ray source controller 23. The X-ray source controller 23 sends the emission start signal, inputted from the emission switch 21, to the X-ray tube 16a through the high voltage generator 22.

The high voltage generator 22 generates the tube voltage and the tube current in accordance with the imaging conditions inputted from the X-ray source controller 23, and applies the tube voltage and the tube current to the X-ray tube 16a. Upon receipt of the emission start signal, the X-ray tube 16a starts X-ray emission in accordance with the tube voltage and tube current applied, and stops the emission when the exposure time has lapsed.

The electronic cassette 11 receives the emission of the X-rays passed through the subject H and detects an X-ray image that carries image information of the subject H. Thereby, the electronic cassette 11 produces image data. The image data is stored in a flash memory 32 of a USB memory device 30 whose access lock has been unlocked as described below.

The console 13 is composed of an operation input section 25 and a notebook computer or the like. The operation input section 25 comprises a keyboard 25a, a mouse 25b, and the like. The notebook computer or the like is integrally provided with a display section 26 for displaying an X-ray image and the like. The display section 26 is composed of a monitor such as an LCD. A housing 27 of the console 13 is provided with a port section 28 to which the USB memory device 30 is attached. The port section 28 has a slot corresponding to an external shape of the memory device 30 and a USB interface (not shown) in the rear of the slot.

In addition, the console 13 is provided with a wireless communicator, an image processor, and the like. The wireless communicator transmits and receives data to and from the electronic cassette 11. The image processor performs various image processes on the image data received from the electronic cassette 11 through the wireless communicator or the USB memory device 30.

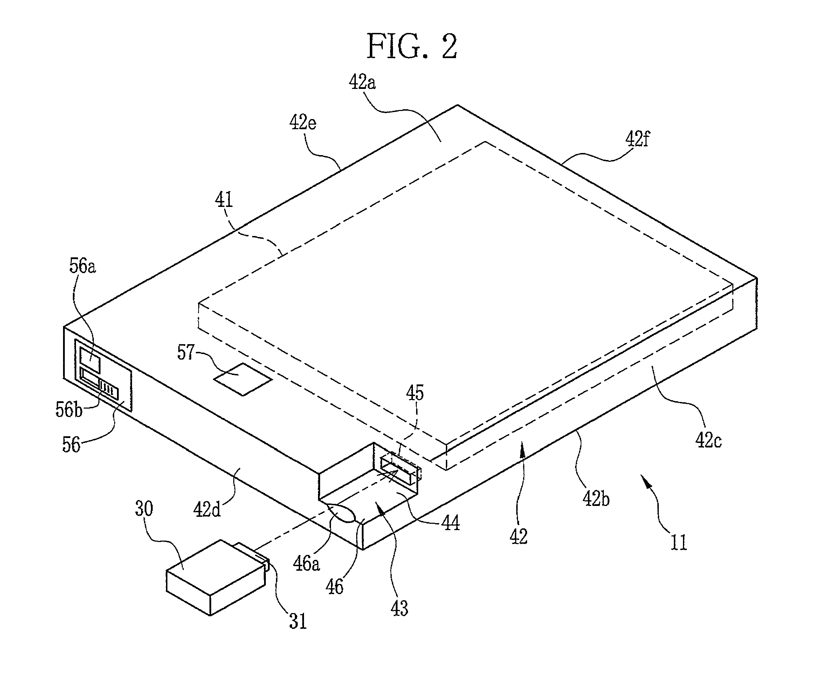

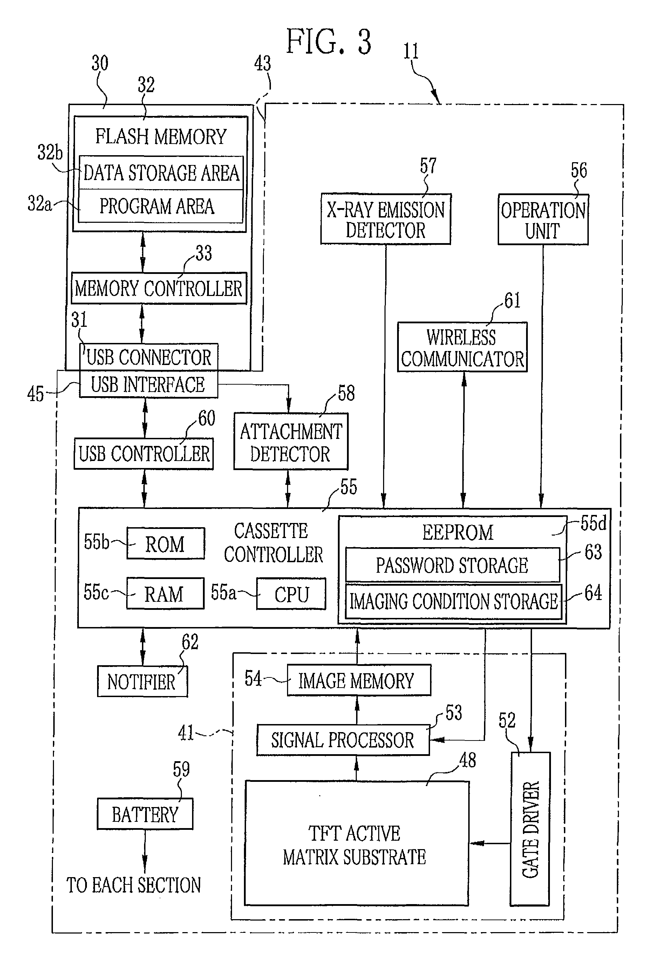

In Figs. 2 and 3, the USB memory device 30, being the universal memory device is substantially rectangular parallelepiped. The USB memory device 30 is provided with a convex-type USB connector 31 that protrudes from an end face of the USB memory device 30. The USB memory device 30 is provided with the flash memory 32, being a nonvolatile memory, and a memory controller 33 for controlling each section of the USB memory device 30. The flash memory 32 has a program area 32a and a data storage area 32b. A program including a password is stored in the program area 32a in advance.

The memory controller 33 places an access lock on the data storage area 32b with the use of the program stored in the program area 32a. When the USB memory device 30 is connected to an external device, the memory controller 33 compares an unlock password inputted from the external device with the password stored in the program area 32a. The memory controller 33 unlocks the access lock only when the unlock password matches the password, and allows writing and reading of data to/from the data storage area 32b. When the passwords match and the access lock is unlocked, the memory controller 33 transmits an unlocking signal for access lock to the external device. When the passwords do not match, the memory controller 33 does not unlock the access lock, and sends an unlock inhibiting signal for access lock to the external device.

The console 13 allows unlocking the access lock on the data storage area 32b in a state that the USB memory device 30 is attached to the port section 28. As a method for unlocking the access lock unlock, the console 13 prompts a user to input the unlock password when the USB memory device 30 is attached to the port section 28, and inputs the unlock password to the USB memory device 30 in accordance with the operation of the operation input section 25 such as the keyboard 25a. The memory controller 33 unlocks the access lock on the data storage area 32b when the unlock password inputted from the operation input section 25 matches the password previously stored in the program area 32a.

The console 13 writes the imaging conditions of X-ray imaging with the electronic cassette 11 to the data storage area 32b of the USB memory device 30. To write the imaging conditions to the data storage area 32b, the access lock on the data storage area 32b is unlocked, similar to reading the image data from the USB memory device 30 attached to the port section 28 of the console 13. Then, the imaging conditions are written to the data storage area 32b whose access lock has been unlocked. Note that the imaging conditions written to the USB memory device 30 by the console 13 include the voltage, the tube current, and the exposure time, which are the same as those inputted to the X-ray source controller 23 through the operation panel of the medical equipment carrier 12.

In Fig. 2, the electronic cassette 11 is provided with an FPD 41, being the image detector, and a housing 42 that houses the FPD 41. A lead plate (not shown) that absorbs back-scattered X-rays is disposed on the back surface side of the FPD 41. A circuit board (not shown) is disposed behind the lead plate.

The housing 42 has a flat, rectangular parallelepiped shape. The housing 42 has a substantially rectangular irradiated area 42a for receiving the emitted radiation, a back surface 42b on the opposite side of the irradiated area 42a, and four lateral surfaces 42c to 42f each along one of four sides of the irradiated area 42a and one of four sides of the back surface 42b. The housing 42 is composed of a case body and a flat plate. The case body is made from stainless steel or aluminum and formed with an opening in an area corresponding to a detection surface (the surface on which the X-rays are incident) of the FPD 41. The flat plate is made from an X-ray transmissive material (for example, carbon) and fitted into the opening of the case body to constitute the irradiated area 42a.

The housing 42 is provided with an attachment portion 43 to which the USB memory device 30 is attached in a detachable manner. The attachment portion 43 is provided on the lateral surface 42d along a short-side direction of the housing 42.

The attachment portion 43 has an accommodation space 44 for accommodating the USB memory device 30 and a USB interface 45 disposed in the accommodation space 44. The USB interface 45 is connected to the USB connector 31. The USB interface 45 is a connector portion having terminals used for electric connection with the USB memory device 30. The USB interface 45 has a concave shape that engages the convex-type USB connector 31. The USB memory device 30 is used with the USB connector 31 connected to the USB interface 45.

The accommodation space 44 is formed with a cutout 46 from which a corner portion where the lateral surfaces 42c and 42d and the irradiated area 42a meet is cut out. A concave portion 46a into which a part of a fingertip is inserted is formed at a periphery of the cutout 46 so as to facilitate holding an edge of the USB memory device 30 when the memory device 30 is removed from the attachment portion 43. Note that the shape of the accommodation space 44 is not limited to this. The accommodation space 44 may have a slot-like shape corresponding to an external shape of the USB memory device 30.

The USB interface 45 is provided in the rear of the accommodation space 44 relative to the lateral surface 42d of the housing 42. The accommodation space 44 has the size enough to accommodate the USB memory device 30 to prevent the USB memory device 30 from projecting to the outside of the housing 42 when the USB connector 31 of the USB memory device 30 is connected to the USB interface 45. The electronic cassette 11 with the USB memory device 30 attached to the attachment portion 43 is referred to as the electronic cassette apparatus.

In Fig. 3, the FPD 41, a cassette controller 55, an operation unit 56, an X-ray emission detector 57, an attachment detector 58, a battery 59, a USB controller 60, a wireless communicator 61, and a notifier 62 are provided inside the housing 42 of the electronic cassette 11. The cassette controller 55 is composed of a microcomputer comprising a CPU 55a, ROM 55b, RAM 55c, EEPROM 55d, and the like. The CPU 55a controls the whole electronic cassette 11. The ROM 55b stores a program which is executed by the CPU 55a. The RAM 55c is a working memory that the CPU 55a uses to execute processing. The EEPROM 55d stores various types of setting information. The operation unit 56 is composed of a power switch 56a, an on/off switch 56b (see Fig. 2) of the wireless communicator 61, and the like.

The USB controller 60 is connected to the cassette controller 55 and the USB interface 45. The USB controller 60 controls writing and reading data to/from the USB memory device 30 when the USB memory device 30 is in an attached state, with the USB connector 31 connected to the USB interface 45. In particular, in this embodiment, the USB controller 60 reads out data of imaging conditions from and writes image data, captured with the electronic cassette 11, to the data storage area 32b of the USB memory device 30 attached to the attachment portion 43 and whose access lock has been unlocked. The USB controller 60 functions as a data reader and a data writer for the USB memory device 30.

The attachment detector 58 detects an output from a signal line of the USB connector 31 through the USB interface 45 to detect whether the USB memory device 30 is attached to the attachment portion 43 or not. The battery 59 provides power to each section in the electronic cassette 11.

In this embodiment, the EEPROM 55d is used as password storage 63 and imaging condition storage 64. The unlock password for unlocking the access lock on the USB memory device 30 is stored in the password storage 63 in advance.

A default password is stored in each of the program area 32a of the USB memory device 30 and the password storage 63 of the electronic cassette 11. The electronic cassette 11 and the USB memory device 30 having the same password are shipped as a set. In this embodiment, one electronic cassette 11 and one USB memory device 30 are used as a set, and the same password is stored in each of them. Hereinafter, the USB memory device 30 with the same password as that stored in the password storage 63 of the electronic cassette 11 in advance is referred to as matching USB memory device 30A. The USB memory device 30 with a password different from that stored in the password storage 63 or the USB memory device 30 with no access lock function is referred to as non-matching USB memory device 30B.

The wireless communicator 61 is a communicator that communicates with the console 13. The wireless communicator 61 is composed of an antenna and a communication circuit that generates radio waves for communication. The cassette controller 55 allows the operation of wireless communicator 61 only when the matching USB memory device 30A is attached to the attachment portion 43. The cassette controller 55 stops the operation of the wireless communicator 61 when the non-matching USB memory device 30B is attached to the attachment portion 43. The cassette controller 55 stops the operation of the wireless communicator 61 when the on/off switch 56b is turned off, regardless of whether the matching USB memory device 30A is attached to the attachment portion 43 or not.

In this embodiment, the electronic cassette 11 is allowed to communicate with the console 13 via the wireless communicator 61 only when the matching USB memory device 30A is attached to the attachment portion 43 and the on/off switch is turned on. Note that the wireless communicator 61 may be an optical communication device using infrared rays or the like.

The default password is stored in each of the program area 32a of the matching USB memory device 30A and the password storage 63 of the electronic cassette 11 at a factory before shipment. The user can change the default password. In this case, for example, the cassette controller 55 and the memory controller 33 function as a password rewriter (password changer). Software for changing password is installed in the console 13. The electronic cassette 11 and the console 13 are capable of wireless communication with each other. Namely, the matching USB memory device 30A is attached to the attachment portion 43 and the on/off switch 56b is turned on. Thereby, the operation of the wireless communicator 61 of the electronic cassette 11 is permitted.

The software for changing password installed in the console 13 is started. Then, each of the current password and a new password is inputted using the keyboard 25a or the like. When the current password inputted to the console 13 matches the password stored in each of the program area 32a of the matching USB memory device 30A and the password storage 63 of the electronic cassette 11, a command for changing the password and the new password are transmitted to the cassette controller 55 of the electronic cassette 11 via the wireless communicator of the console 13. The command for changing the password and the new password are transmitted from the cassette controller 55 to the matching USB memory device 30A via the USB controller 60 and the USB interface 45. Upon receiving the command for changing the password and the new password from the console 13, the cassette controller 55 stores the new password in the password storage 63 and the memory controller 33 stores the new password in the program area 32a. Thus, the passwords are overwritten simultaneously.

As described above, the operation of the wireless communicator 61 is permitted when the matching USB memory device 30A is attached to the attachment portion 43. Hence, the password of the electronic cassette 11 is not changed when the matching USB memory device 30A is not attached. In other words, the above-described operation for changing the password does not make the passwords of the electronic cassette 11 and the matching USB memory device 30A different from each other.

Imaging conditions necessary for X-ray imaging using the electronic cassette 11 are stored in the imaging condition storage 64 of the EEPROM 55d. When the USB memory device 30 is attached to the attachment portion 43, the cassette controller 55 receives the imaging conditions written to the data storage area 32b of the USB memory device 30 by the console 13, and allows the imaging condition storage 64 to store the imaging conditions.

The cassette controller 55 functions as an unlock password input section and a memory determining section based on the program running from the ROM 55b. The cassette controller 55 functions as the unlock password input section when the attachment detector 58 detects that the USB memory device 30 is attached to the attachment portion 43. The cassette controller 55, being the unlock password input section, controls the USB controller 60 and inputs the unlock password, stored in the password storage 63, to the USB memory device 30. When the matching USB memory device 30A is attached to the attachment portion 43, the unlock password inputted from the cassette controller 55 matches the password stored in the program area 32a. Thereby, the access lock on the data storage area 32b is unlocked.

The cassette controller 55 also functions as the memory determining section to determine whether the USB memory device 30 attached to the attachment portion 43 is the matching USB memory device 30A or the non-matching USB memory device 30B. To be more specific, the cassette controller 55, being the unlock password input section, inputs the unlock password to the USB memory device 30. When receiving the unlocking signal for access lock from the USB memory device 30, the cassette controller 55 determines that the USB memory device 30 attached to the attachment portion 43 is the matching USB memory device 30A. When not receiving the unlocking signal for access lock, the cassette controller 55 determines that the USB memory device 30 attached to the attachment portion 43 is the non-matching USB memory device 30B. The latter case, "when not receiving the unlocking signal for access lock", includes the case where an unlock inhibiting signal for access lock is received due to the attachment of the non-matching USB memory device 30B set with a different password and the case where no signal is received due to the attachment of the non-matching USB memory device 30B with no access lock function working with a password.

When the cassette controller 55 determines that the USB memory device 30 attached to the attachment portion 43 is the matching USB memory device 30A, the cassette controller 55 allows writing of image data. When the cassette controller 55 determines that the USB memory device 30 attached is the non-matching USB memory device 30B, the cassette controller 55 inhibits writing of image data.

The X-ray emission detector 57 is composed of an X-ray detection element that absorbs the X-rays and converts the X-rays into charge. The X-ray emission detector 57 is provided close to the irradiated area 42a of the housing 42. The X-ray emission detector 57 detects X-ray emission on the irradiated area 42a and sends a detection signal to the cassette controller 55. When the X-ray emission detector 57 detects the X-ray emission on the irradiated area 42a, the cassette controller 55 allows the FPD 41 to perform image detection operation based on the imaging conditions stored in the imaging condition storage 64 of the EEPROM 55d. The FPD 41 temporarily stores the image data, obtained by the image detection operation, in an image memory 54. The cassette controller 55 controls the USB controller 60 to allow writing of image data, read from the image memory 54, to the data storage area 32b of the matching USB memory device 30A with the access lock unlocked.

The notifier 62 is composed of an LED lamp, a speaker that outputs buzzing sound, or the like. The notifier 62 notifies various types of error messages. The notifier 62 may be provided with LED lamps and turn on an LED lamp according to the type of the error. The notifier 62 may change a rhythm of the buzzing sound according to the type of the error. The error messages include those related to the USB memory device 30, for example, an error message indicating that the USB memory device is not attached and an error message indicating that the non-matching USB memory device 30B is attached.

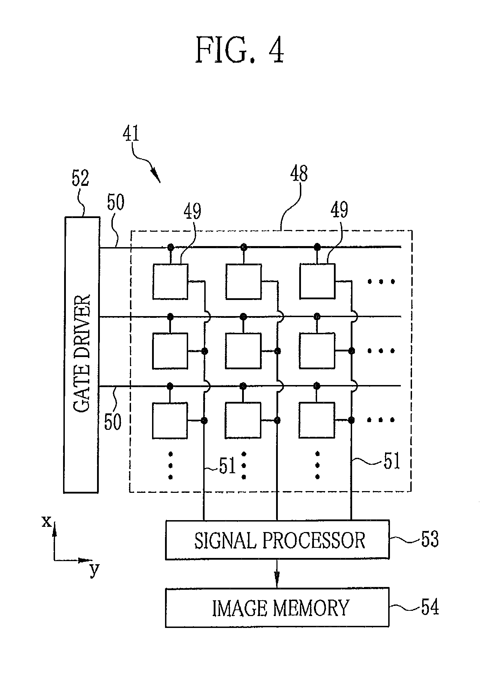

In Fig. 4, in the FPD 41, a charge generating layer (not shown) that absorbs X-rays and converts the X-rays into charge is provided over a TFT active matrix substrate 48, as is well known. The charge generating layer is made from, for example, amorphous selenium (a-Se) having selenium as a main component (for example, with a content of 50% or more). When the X-rays are emitted, the charge (electron-hole pairs) with an amount corresponding to an amount of the incident X-rays is generated in the charge generating layer. Thus, the charge generating layer converts the emitted X-rays into the charge. The FPD 41 may be an indirect conversion type that converts the X-rays into visible light with a scintillator and then converts the visible light into the charge.

As is well known, a plurality of capacitors and a plurality of pixels 49 are arranged in matrix over the TFT active matrix substrate 48. Each capacitor stores the charge generated in the charge generating layer. Each pixel 49 is provided with a TFT for reading the charge stored in the capacitor. Each pixel 49 detects the incident X-rays. The charge, which is generated in the charge generating layer in accordance with the amount of the X-rays incident on each pixel 49, is stored in the capacitor. Thereby, the image information of the subject H carried by the X-rays incident on the irradiated area 42a is converted into charge information and held by the TFT active matrix substrate 48.

The TFT active matrix substrate 48 is provided with gate lines 50 extending in a row direction and data lines 51 extending in a column direction orthogonal to the gate lines 50. The gate line 50 is used for turning on and off gates of the TFTs of the respective pixels 49. The data line 51 is used for reading the stored charge from the capacitor through the turned on TFT. The gate lines 50 are connected to a gate driver 52. The data lines 51 are connected to a signal processor 53. When the charge is stored in capacitors of the respective pixels 49, a signal supplied from the gate driver 52 through the gate line 50 turns on the TFTs of the corresponding pixels 49 on a row-by-row basis sequentially. The charge stored in the capacitor of the pixel 49 with the TFT turned on is transmitted as a charge signal through the data line 51 and inputted to the signal processor 53.

The signal processor 53 is provided with amplifiers and sample hold circuits. The amplifier and the sample hold circuit are provided to each of the data lines 51. The charge signal transmitted through each data line 51 is amplified in the amplifier and then held by the sample hold circuit. A multiplexer and an A/D converter are connected in this order on an output side of the sample hold circuit. The charge signal held by each sample hold circuit is inputted to the multiplexer sequentially (serially) and then converted into digital image data by the A/D converter. The image data outputted from the A/D converter of the signal processor 53 is stored in the image memory 54 sequentially. The image memory 54 has a memory capacity capable of storing image data of images.

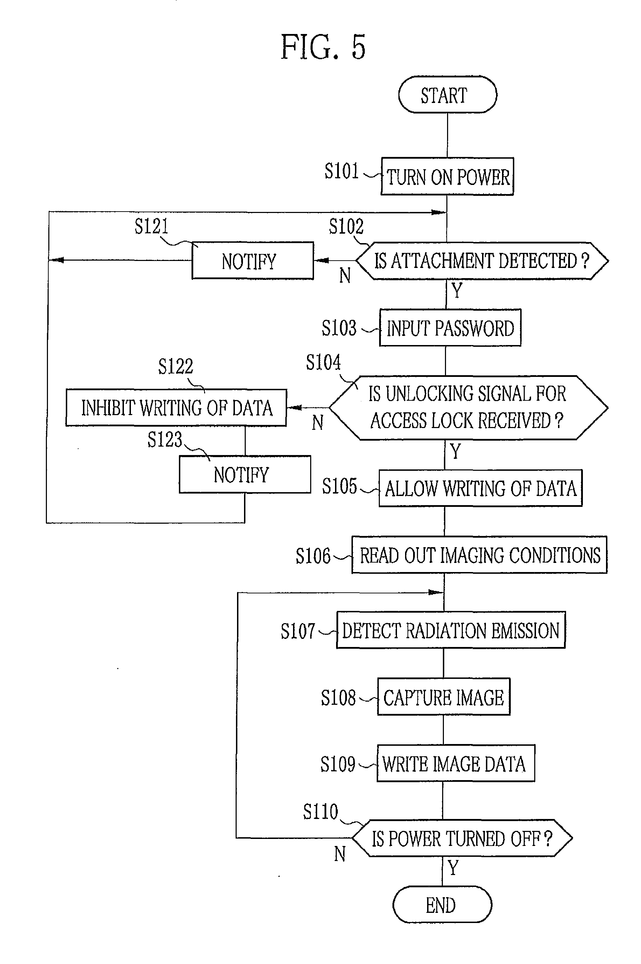

Next, referring to a flowchart in Fig. 5, the operation of the above configuration is described. To perform X-ray imaging using the electronic cassette 11, first, the power switch 56a is operated to turn on power (S101). When the power of the electronic cassette 11 is turned on, the cassette controller 55 is activated and starts controlling each section. At this time, the wireless communicator 61 is turned off by the on/off switch 56b.

The activated cassette controller 55 allows the attachment detector 58 to detect whether the USB memory device 30 is attached to the attachment portion 43 or not (S102). Note that when the attachment of the USB memory device 30 is not detected (N in S102), the cassette controller 55 activates the notifier 62 to notify an error message indicating that the USB memory device 30 is not attached and prompts attachment of the USB memory device 30 (S121).

Upon detecting that the USB memory device 30 is attached to the attachment portion 43 (Y in S102), the cassette controller 55 functions as the unlock password input section. The cassette controller 55 reads the unlock password stored in the password storage 63, and controls the USB controller 60 to input the unlock password to the USB memory device 30 (S103).

After functioning as the unlock password input section and inputting the unlock password (S103), the cassette controller 55 functions as the memory determining section. When the matching USB memory device 30A is attached to the attachment portion 43, the unlock password inputted by the cassette controller 55 matches the password stored in the program area 32a of the matching USB memory device 30A. In the matching USB memory device 30A, the memory controller 33 unlocks the access lock on the data storage area 32b and transmits the unlocking signal for access lock. The cassette controller 55 receives the unlocking signal for access lock (Y in S104). Upon receiving the unlocking signal for access lock, the cassette controller 55 determines that the USB memory device 30 attached to the attachment portion 43 is the matching USB memory device 30A and allows writing of the image data (S105).

When the non-matching USB memory device 30B is attached to the attachment portion 43, the cassette controller 55 does not receive the unlocking signal for access lock (N in S104). As described above, the unlocking signal for access lock is not outputted when the non-matching USB memory device 30B with the different password is attached and when the non-matching USB memory device 30B with no access lock function is attached. In this case, the cassette controller 55 determines that the USB memory device 30 attached to the attachment portion 43 is the non-matching USB memory device 30B. Thereby, the cassette controller 55 inhibits writing of image data (S122) and activates the notifier 62. The notifier 62 notifies the error message indicating that the non-matching USB memory device 30B is attached (S123).

After the cassette controller 55 determines that the USB memory device 30 attached to the attachment portion 43 is the matching USB memory device 30A and allows writing of image data (S105), the cassette controller 55 reads the imaging conditions stored in the data storage area 32b and stores the imaging conditions in the imaging condition storage 64 of the EEPROM 55d (S106).

When the X-ray emission detector 57 detects the X-ray emission on the irradiated area 42a (S107) after the cassette controller 55 reads and stores the imaging conditions, the cassette controller 55 allows the FPD 41 to perform the image detection operation based on the imaging conditions stored in the imaging condition storage 64. Thereby, the X-ray imaging is performed. The image data is temporarily stored in the image memory 54 (S108).

At this time, the matching USB memory device 30A is in a state in which writing of image data is permitted and the access lock on the data storage area 32b is unlocked. Thereby, the USB controller 60 writes the image data, temporarily stored in the image memory 54, to the matching data storage area 32b (S109). When the non-matching USB memory device 30B is attached, on the other hand, writing of image data is inhibited. Note that, in this case, the X-ray imaging may be inhibited.

When the imaging is completed and the power of the electronic cassette 11 is turned off (Y in S110), the matching USB memory device 30A is removed from the attachment portion 43. To continue the imaging, the power of the electronic cassette 11 is kept turned on (N in S110) and the matching USB memory device 30A is kept attached. When the X-ray emission detector 57 detects the X-ray emission again (S107), the X-ray imaging is performed (S108). The matching USB memory device 30A is in a state in which writing of image data is permitted and the access lock on the data storage area 32b is unlocked. Thereby, the image data is written to the data storage area 32b (S110). The imaging is repeated as described above until the power of the electronic cassette 11 is turned off (Y in S111).

The matching USB memory device 30A removed from the electronic cassette 11 is attached to the port section 28 of the console 13. The USB connector 31 is connected to the USB interface of the console 13. As described above, the console 13 unlocks the access lock on the data storage area 32b with the password inputted through the operation input section 25, and permits reading of image data. This allows the USB controller to read the image data from the data storage area 32b. Image processing is performed on the image data and an X-ray image is displayed on the display section 26.

As described above, in the electronic cassette 11, the image data is stored in the USB memory device 30 in which the access lock is placed on the data storage area 32b. Hence, leakage of image data to the outside world is prevented even if the USB memory device 30 is lost. When the matching USB memory device 30A is attached to the attachment portion 43 of the electronic cassette 11, the cassette controller 55 inputs the unlock password to the matching USB memory device 30A and the access lock on the data storage area 32b is unlocked. The user does not need to perform bothersome operation such as manually inputting a password during preparation for imaging. Thus, usability of the electronic cassette 11 is improved.

There are cases where the shape of the USB connector 31 of the non-matching USB memory device 30B is the same as that of the matching USB memory device 30, so that the USB connector 31 of the non-matching USB memory device 30B can be attached to the attachment portion 43 of the electronic cassette 11. In this embodiment, it is determined whether the USB memory device 30 attached to the attachment portion 43 is the matching USB memory device 30A or the non-matching USB memory device 30B. Writing the data to the matching USB memory device 30A is permitted. Writing the data to the non-matching USB memory device 30B is inhibited. Thus, writing the data to the non-matching USB memory device 30B not corresponding to the electronic cassette 11 (the non-matching USB memory device 30B having a different password) or the non-matching USB memory device 30B not having the access lock function is inhibited. This surely prevents the leakage of image data to the outside world.

Image data cannot be obtained from the electronic cassette 11 unless the matching USB memory device 30A is used. A user with no matching USB memory device 30A cannot use the electronic cassette 11. This prevents unauthorized use of the electronic cassette 11, for example, theft.

Note that, instead of transmitting the imaging conditions from the console 13 to the electronic cassette 11 through the matching USB memory device 30A as described in the above embodiments, the electronic cassette 11 and the console 13 may be moved to a location where the wireless communication is possible only at the time of inputting the imaging conditions. The wireless communicator 61 of the electronic cassette 11 and the wireless communicator of the console 13 are turned on, and data of the imaging conditions is transmitted from the console 13 to the electronic cassette 11 through the wireless communication.

In the above embodiments, the console 13 communicates with the electronic cassette 11 through wireless communication. The present invention is not limited to this. A communication section for cable communication may be provided. The communication section connects the console 13 and the electronic cassette 11 via a cable. In this case, if the cable used for the cable communication is in the way during positioning of the subject H, for example, the cable communication is turned off and the cable is removed. The image data is stored using the matching USB memory device 30A.

In the above embodiments, the single electronic cassette 11 and the single matching USB memory device 30A are used as a set. The same password is stored in each of the electronic cassette 11 and the matching USB memory device 30A. The present invention is not limited to this. For example, the electronic cassettes 11 and the single matching USB memory device 30A may be used as a set. The same password is stored in each of the electronic cassettes 11 and the matching USB memory device 30A. Alternatively, the single electronic cassette 11 and the matching USB memory devices 30A may be used as a set. In this case, different passwords are assigned to the matching USB memory devices 30A, respectively. The unlock passwords corresponding to the respective matching USB memory devices 30A are stored in the password storage 63 of the electronic cassette 11.

In the above embodiments, the USB memory device is used as the universal memory device. The universal memory device is not limited to this. Another universal memory device, for example, which is not compliant with the USB standard such as an SD card may be used.

In the above embodiments, the X-rays are used as the radiation by way of example. In the present invention, radiation other than the X-rays, for example, gamma rays may be used. The present invention is not limited to the above embodiments, and various changes are possible within the subject matter of the present invention.

## Claims

1. An electronic cassette to which a memory device is attached in a detachable manner, writing to the memory device being allowed with an access lock unlocked by inputting an unlock password, the electronic cassette comprising:
an image detector for detecting radiation passed through a subject and producing image data;
an unlock password storage for storing the unlock password; and
an unlock password input section for reading the unlock password from the unlock password storage and inputting the unlock password to the memory device after the memory device is attached.

2. The electronic cassette of claim 1 further comprising:
a controller for determining the memory device, with the access lock unlocked by inputting the unlock password, as a matching memory device and allowing writing of the image data, the controller inhibiting writing of the image data to a non-matching memory device that is not the matching memory device.

3. The electronic cassette of claim 1 further comprising:
a password changer for changing the unlock password, stored in the unlock password storage, based on a change command inputted from an external device.

4. The electronic cassette of claim 1 further comprising:
a data reader for reading data, related to imaging conditions of radiation imaging, from the matching memory device.

5. The electronic cassette of claim 2, further comprising:
a communicator for communicating data with an external device.

6. The electronic cassette of claim 5, wherein the controller allows the communicator to operate only when the matching memory device is attached to the memory attachment portion.

7. The electronic cassette of claim 1, wherein the memory device is a USB memory device.

8. An electronic cassette apparatus comprising:
a memory device to which writing is allowed with an access lock unlocked by inputting an unlock password;
a memory attachment portion to which the memory device is attached in a detachable manner;
an image detector for detecting radiation passed through a subject and producing image data;
an unlock password storage for storing the unlock password; and
an unlock password input section for reading the unlock password from the unlock password storage and inputting the unlock password to the memory device after the memory device is attached to the memory attachment portion.
